# EUROPÄISCHE PATENTSCHRIFT

(11) **EP 2 063 797 B1**
(45) Veröffentlichungstag und Bekanntmachung des Hinweises auf die Patenterteilung: **19.10.2011**
(21) Anmeldenummer: 07786520.2
(22) Anmeldetag: 02.08.2007
(51) Int. Cl.: A61B 17/82, A61B 17/68, A61B 17/80

(54) **STERNUMVERSCHLUSS**
STERNUM CLOSURE DEVICE
DISPOSITIF DE FERMETURE STERNALE

(30) Priorität: 22.09.2006 DE 102006046428
(43) Veröffentlichungstag der Anmeldung: 03.06.2009
(73) Patentinhaber: Aesculap AG, 78532 Tuttlingen (DE)
(72) Erfinder: MORALES, Pedro, 78532 Tuttlingen (DE); WEISSHAUPT, Dieter, 78194 Immendingen (DE); LUTZE, Theodor, 78582 Balgheim (DE); DWORSCHAK, Manfred, 78589 Dürbheim (DE); EIJSMAN, Leon, NL-1261 AJ Blaricum (NL)
(74) Vertreter: Hoeger, Stellrecht & Partner Patentanwälte
(86) Internationale Anmeldenummer: PCT/EP2007/006846
(87) Internationale Veröffentlichungsnummer: WO 2008/034489

(56) Entgegenhaltungen:
- WO-A-2004/006783
- WO-A-2004/016205
- WO-A-2004/107998
- DE-U1- 29 919 090
- US-A- 5 549 620
- US-A1- 2005 065 521
- US-A1- 2005 278 027

## Beschreibung

Die Erfindung betrifft einen Sternumverschluss zur Festlegung von zwei miteinander zu verbindenden Sternumteilen mit einem inneren Anlageelement zur Anlage an der Innenfläche des Sternums, mindestens einem daran festgelegten, quer von diesem abstehenden Spannelement und mit einem äußeren Anlageelement zur Anlage an der Außenseite des Sternums, welches mittels des durch den Zwischenraum zwischen den Sternumteilen hindurchgeführten Spannelements gegen das erste Anlageelement spannbar ist, wobei das innere Anlageelement zumindest teilweise aus einem körperverträglichen Kunststoff besteht und wobei in den Kunststoff Metallteile eingebettet sind.

Mit Hilfe eines Sternumverschlusses können die beiden durch einen Trennschnitt voneinander getrennten Sternumteile nach einer Operation so gegeneinander gespannt und relativ zueinander fixiert werden, dass in einem Heilungsprozess knöcherne Substanz gebildet wird, die die beiden Sternumteile wieder verbindet. Die Teile des Sternumverschlusses, die regelmäßig aus einem körperverträglichen Metall bestehen, insbesondere aus Titan oder einer Titanlegierung, verbleiben im Körper (WO2004/006783 A1; DE 103 26 690 B4). In der WO2004/006783 A1 ist auch beschrieben, dass das innere Anlageelement des Sternumverschlusses aus Kunststoff bestehen kann und dass gegebenenfalls Teile des Sternumverschlusses, die eine größere Festigkeit erfordern, aus Metall bestehen können, welches in das absorbierbare Kunststoffmaterial eingesetzt ist.

Falls eine erneute Öffnung des Sternums mit Trennung desselben in zwei Sternumteile notwendig wird, behindern allerdings die Teile des Sternumverschlusses möglicherweise den Trennvorgang. Dies gilt insbesondere für die an der Innenfläche des Sternums anliegenden inneren Anlageelemente, die nicht ohne weiteres entfernt werden können. Bei den äußeren Anlageelementen besteht die Möglichkeit, diese abzuheben, bei den inneren Anlageelementen jedoch fehlt die Möglichkeit eines Zugangs und daher ist es schwierig, das Sternum mit einer Knochensäge zu durchtrennen.

Es ist Aufgabe der Erfindung, einen gattungsgemäßen Sternumverschluss so auszubilden, dass ein Durchtrennen des Sternums bei einer erneuten Operation erleichtert wird.

Diese Aufgabe wird bei einem Sternumverschluss der eingangs beschriebenen Art erfindungsgemäß dadurch gelöst, dass das innere Anlageelement ein Mittelteil aus dem körperverträglichen Kunststoff aufweist, in den auf beiden gegenüberliegenden Längsseiten jeweils ein mit Haltevorsprüngen zur Fixierung des inneren Anlageelementes am Sternum versehener, leistenförmiger Steg aus Metall eingebettet ist.

Ein inneres Anlageelement, das in seinem Mittelteil aus einem körperverträglichen Kunststoff besteht, kann beim Durchtrennen des Sternums ebenfalls durchtrennt werden, sei es mit der Knochensäge, sei es nach Einbringen eines Knochentrennschnittes mit einer Schere oder einem ähnlichen Instrument. Nach der Trennung des inneren Anlageelementes können die Sternumteile ohne weiteres aufgespreizt werden, so dass ein Körperzugang geschaffen wird.

Die Haltevorsprünge werden beim Andrücken des inneren Halteelementes an die Innenfläche des Sternums in die Knochensubstanz eingedrückt und fixieren dadurch das innere Anlageelement an der Innenfläche des Sternums. Diese Haltevorsprünge sollten vorzugsweise aus Metall bestehen, da sie große Kräfte ausüben müssen. Es ist daher vorteilhaft, wenn das innere Anlageelement zwar einerseits Haltevorsprünge aus Metall aufweist, andererseits aber ein Mittelteil aus Kunststoff, das in der beschriebenen Weise bei einer Reoperation wieder durchtrennt werden kann.

Als Kunststoff kommen beispielsweise in Frage Polyetheretherketon, Polyethylen oder Polyamid oder insbesondere auch resorbierbare Kunststoffe, beispielsweise Polylactid oder ein Polylactidcopolymer.

Die Einbettung der leistenförmigen Stege kann beispielsweise durch Umspritzen der Haltevorsprünge mit Kunststoffmaterial erfolgen.

Günstig ist es, wenn die Haltevorsprünge und die leistenförmigen Stege einstückig ausgebildet sind.

Gemäß einer bevorzugten Ausführungsform ist vorgesehen, dass der Steg Durchbrechungen aufweist, durch die der Kunststoff hindurchtritt, so dass eine innige Verbindung zwischen dem Steg einerseits und dem Kunststoffmaterial andererseits erreicht wird.

Insbesondere kann vorgesehen sein, dass die Haltevorsprünge in zwei auf gegenüberliegenden Seiten des Mittelteils verlaufenden Reihen angeordnet sind.

Die nachfolgende Beschreibung bevorzugter Ausführungsformen der Erfindung dient im Zusammenhang mit der Zeichnung der näheren Erläuterung. Es zeigen:
- Figur 1 :: eine perspektivische Ansicht eines ersten bevorzugten Ausführungsbeispiels eines Sternumverschlusses mit einem inneren Anlageelement mit einem Mittelteil aus Kunststoff und Haltevorsprüngen aus Metall vor dem Spannen der Anlageelemente;
- Figur 2 :: eine weitere perspektivische Ansicht des Sternumverschlusses der Figur 1 in einer Ansicht schräg von unten und
- Figur 3 :: eine perspektivische Ansicht der Einzelteile des inneren Anlageelementes in einer Explosionsdarstellung.

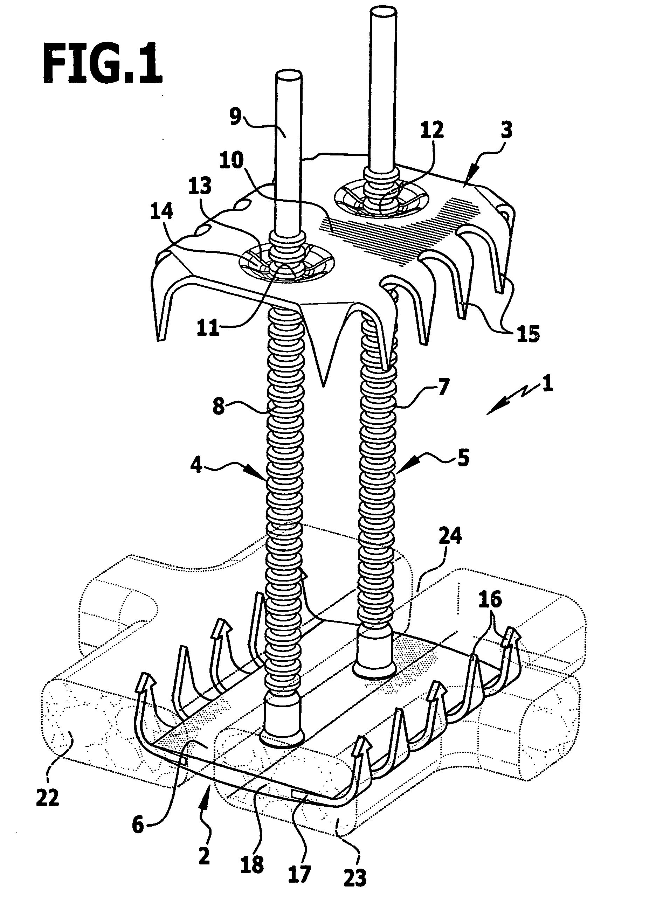

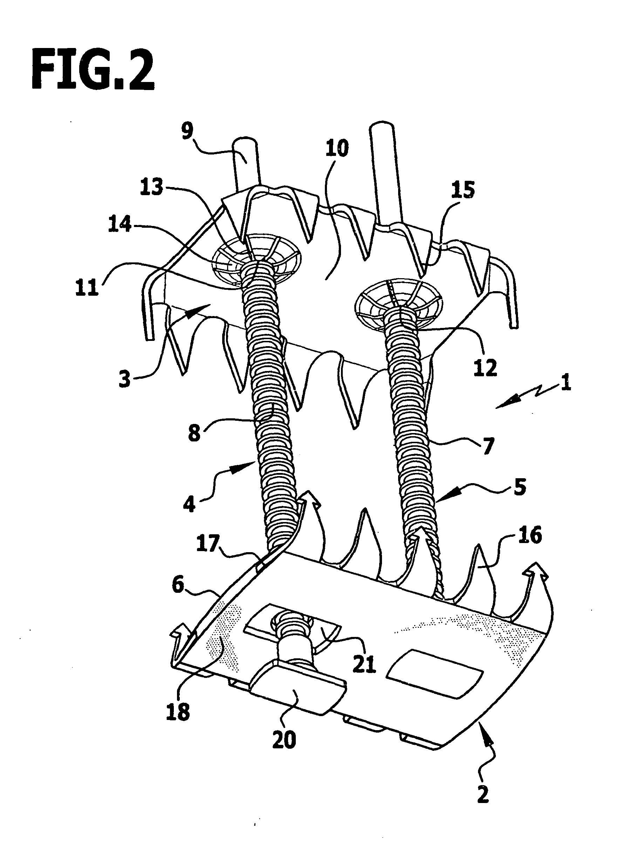

Der in den Figuren 1 bis 3 dargestellte Sternumverschluss 1 weist ein inneres Anlageelement 2 und ein äußeres Anlageelement 3 auf, die über zwei als Spannelement wirkende Raststifte 4, 5 miteinander verbunden sind und gegeneinander gespannt werden können. Die Raststifte 4,5 sind an dem plattenförmigen, im dargestellten Ausführungsbeispiel im wesentlichen rechteckigen inneren Anlageelement 2 so gehalten, dass sie senkrecht von der Oberseite des inneren Anlageelementes 2 abstehen, diese Oberseite bildet eine im wesentlichen ebene Anlagefläche 6 aus.

Die beiden Raststifte 4,5 tragen oberhalb des inneren Anlageelementes 2 eine größere Anzahl von Umfangsrippen 7, sie bilden mit diesen einen Rastabschnitt 8 aus, an den sich zum freien Ende der Raststifte 4, 5 hin jeweils ein Verlängerungsabschnitt 9 mit glatter Außenwand anschließt. Auf die Verlängerungsabschnitte 9 der beiden Raststifte 4,5 ist das äußere Anlageelement 3 aufgesteckt, dieses besteht aus Metall und weist ein etwa rechteckförmiges, plattenförmiges Mittelstück 10 mit einer ebenen Unterseite auf und zwei das Mittelstück 10 durchsetzende Öffnungen 11, 12, durch die der Verlängerungsabschnitt 9 der beiden Raststifte 4,5 hindurchragt. Von den Öffnungen 11, 12 gehen radiale Einschnitte 13 aus, die den Randbereich der Öffnungen 11, 12 in voneinander getrennte Lappen 14 unterteilen, diese können elastisch aus ihrer Ausgangslage verbogen werden, wenn das äußere Anlageelement 3 in Richtung auf das innere Anlageelement 2 verschoben wird und die Lappen 14 dabei an den Umfangsrippen 7 entlanggleiten. Dadurch lässt sich das äußere Anlageelement 3 zwar in Richtung auf das innere Anlageelement 2 verschieben, nicht aber in umgekehrter Richtung.

An beiden Längskanten des Mittelstückes 10 ist das Material des äußeren Anlageelementes 3 in Richtung auf das innere Anlageelement 2 etwa rechtwinklig abgebogen und bildet dort eine Reihe von spitzen Haltevorsprüngen 15 aus.

Auch das innere Anlageelement 2 trägt an seinen beiden Längsseiten in Richtung auf das äußere Anlageelement 3 weisende spitze Haltevorsprünge 16, diese Haltevorsprünge 16 sind jeweils auf einer Seite des Mittelstückes 10 über einen Steg 17 miteinander verbunden, der mit den Haltevorsprüngen 16 einstückig ausgebildet ist und aus Metall besteht, beispielsweise aus Titan oder eine Titanlegierung. Der Steg 17 verläuft dabei rechtwinklig zur Richtung der Haltevorsprünge 16 und liegt in der Ebene der Anlagefläche 6 des inneren Anlageelementes 2.

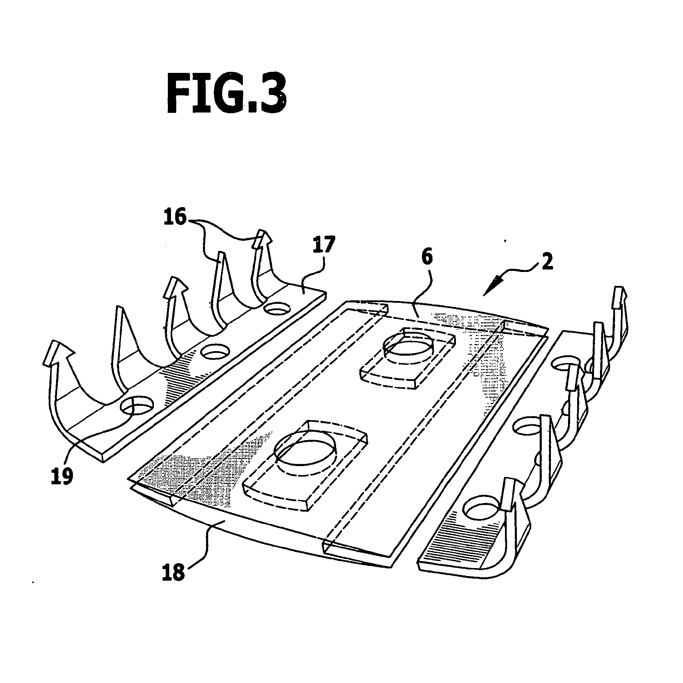

Dieses innere Anlageelement 2 ist mit Ausnahme der Stege 17 und der Haltevorsprünge 16 aus Kunststoff hergestellt, entweder aus einem körperverträglichen dauerhaften Kunststoff wie Polyetheretherketon, Polyethylen oder Polyamid oder aus einem resorbierbaren Kunststoffmaterial, wie beispielsweise Polylactid oder einem Polylactidcopolymer. Dabei ist die Anlagefläche 6 Teil einer Kunststoffplatte 18, die an ihren beiden Längskanten mit dem Steg verbunden ist, beispielsweise dadurch, dass der Steg von dem Material der Kunststoffplatte 18 umspritzt ist. Damit ist der Steg 17 in das Kunststoffmaterial eingebettet und dauerhaft mit ihm verbunden. Die Verbindung kann dadurch besonders dauerhaft ausgebildet sein, dass im Steg 17 Durchbrechungen 19 vorgesehen sind, durch die das Kunststoffmaterial hindurchtritt (Figur 3).

Das innere Anlageelement 2 bildet somit ein zusammengesetztes Bauteil aus, welches neben der zentralen Kunststoffplatte 18 auf beiden gegenüberliegenden Längsseiten jeweils einen mit Haltevorsprüngen 16 versehenen Steg 17 aus Metall umfasst. Durch diesen Steg wird die Kunststoffplatte versteift und stabilisiert, so dass die Haltevorsprünge 16 die nötige Stabilität bekommen, die sie bei der Anlage des inneren Anlageelementes 2 an der Innenseite eines Sternums benötigen.

Die beiden Raststifte 4, 5 tragen an ihrem unteren Ende einen vergrößerten Kopf 20, der in eine komplementäre Vertiefung 21 der Kunststoffplatte eingreift, Kopf und Vertiefung sind im wesentlichen rechteckig ausgebildet, so dass der Raststift unverdrehbar an dem inneren Anlageelement 2 gehalten ist. Grundsätzlich ist es auch möglich, den Kopf 20 mit dem Kunststoffmaterial zu umgeben und ihn vollständig einzubetten, um eine möglichst gute Verbindung herzustellen.

Der Sternumverschluss 1 wird in ähnlicher Weise angelegt wie ein herkömmlicher Sternumverschluss mit einem metallischen inneren Anlageelement 2. Die Anlage erfolgt an die Innenfläche von zwei durch einen Trennschnitt voneinander getrennten und aufgespreizten Sternumteilen 22 und 23. Durch den durch die Aufspreizung der Sternumteile 22, 23 aufgeweiteten Zwischenraum 24 zwischen diesen wird das innere Anlageelement 2 in den Brustraum eingeführt und dann werden nach dem Zusammenspannen der beiden Sternumteile 22, 23 die Haltevorsprünge 16 des inneren Anlageelementes 2 in die Innenfläche der Sternumteile 22, 23 eingedrückt, indem das innere Anlageelement 2 durch Zug an den Raststiften 4, 5 gegen die Innenseite der Sternumteile 22, 23 gespannt wird. Durch ein in der Zeichnung nicht dargestelltes Spanninstrument wird dann das äußere Anlageelement 3 auf den Raststiften 4, 5 in Richtung auf das innere Anlageelement 2 verschoben, bis die Haltevorsprünge 15 von der Außenseite der Sternumteile 22, 23 in diese eingedrungen sind und bis die Oberseite des inneren Anlageelementes 2 und die Unterseite des äußeren Anlageelementes 3 beidseitig am Sternum anliegen und dadurch die beiden Sternumteile 22, 23 dauerhaft relativ zueinander fixieren. In diesem angelegten Zustand können die überstehenden Teile der Raststifte 4, 5 entfernt werden.

Über die Länge des Zwischenraums 24 können mehrere derartige Sternumverschlüsse 1 im Abstand zueinander angelegt werden, so dass über die gesamte Länge des Trennschnittes zwischen den beiden Sternumteilen 22, 23 eine Fixierung dieser beiden Sternumteile 22, 23 gegeneinander erfolgt.

Bei einer eventuell notwendig werdenden erneuten Trennung des Sternums werden zunächst die äußeren Anlageelemente 3 entfernt. Dann kann mit einer Knochensäge entlang den Raststiften 4, 5 eine Durchtrennung des Knochenmaterials des Sternums erfolgen. Bei dieser Durchtrennung kann entweder das Kunststoffmaterial des inneren Anlageelementes 2 ebenfalls durchtrennt werden, oder aber dieses Kunststoffmaterial wird mit einem eigenen Werkzeug durchtrennt, beispielsweise mit einer Schere oder einer Schneidzange. Sobald das innere Anlageelement 2 in zwei Teile getrennt ist, können die Sternumteile 22 und 23 ohne weiteres aufgespreizt werden, und dann ist es auch möglich, die Teile des inneren Anlageelementes 2 einzeln von dem jeweiligen Sternumteil abzuheben und aus der Sternumhinterwand zu entfernen.

Bei dem Ausführungsbeispiel der Figuren 1 bis 3 ist eine Hybridbauweise verwendet worden mit einem inneren Anlageelement 2, das aus einem zentralen Mittelteil aus Kunststoff und aus metallischen Haltevorsprüngen besteht.

## Patentansprüche

1. Sternumverschluss (1) zur Festlegung von zwei miteinander zu verbindenden Sternumteilen (22, 23) mit einem inneren Anlageelement (2) zur Anlage an der Innenfläche des Sternums, mindestens einem daran festgelegten, quer von diesem abstehenden Spannelement und mit einem äußeren Anlageelement (3) zur Anlage an der Außenseite des Sternums, welches mittels des durch den Zwischenraum (24) zwischen den Sternumteilen (22, 23) hindurchgeführten Spannelements gegen das erste Anlageelement spannbar ist, wobei das innere Anlageelement (2) zumindest teilweise aus einem körperverträglichen Kunststoff besteht und wobei in den Kunststoff Metallteile eingebettet sind, **dadurch gekennzeichnet, dass** das innere Anlageelement (2) ein Mittelteil (18) aus dem körperverträglichen Kunststoff aufweist, in den auf beiden gegenüberliegenden Längsseiten jeweils ein mit Haltevorsprüngen (16) zur Fixierung des inneren Anlageelementes (2) am Sternum versehener, leistenförmiger Steg (17) aus Metall eingebettet ist.

2. Sternumverschluss nach Anspruch 1, **dadurch gekennzeichnet, dass** der Kunststoff Polyetheretherketon ist.

3. Sternumverschluss nach Anspruch 1, **dadurch gekennzeichnet, dass** der Kunststoff ein resorbierbarer Kunststoff ist.

4. Sternumverschluss nach einem der voranstehenden Ansprüche, **dadurch gekennzeichnet, dass** der Steg (17) Durchbrechungen (19) aufweist, durch die der Kunststoff hindurchtritt.

## Claims

1. Sternum closure device (1) for securing two sternum parts (22, 23) to be connected to one another, comprising an inner contact element (2) to abut the inner face of the sternum, at least one clamping element secured thereto and projecting transversely therefrom, and comprising an outer contact element (3) for abutment on the outer side of the sternum and which can be clamped by means of the clamping element guided through the intermediate space (24) between the sternum parts (22, 23) against the first contact element, wherein the inner contact element (2) consists at least partially of a biocompatible plastics material and wherein metal parts are embedded in the plastics material, **characterised in that** the inner contact element (2) has a centre part (18) made of the biocompatible plastics material in which on the two opposing longitudinal sides in each a strip-like web (17) made of metal and provided with holding projections (16) to secure the inner contact element (2) on the sternum is embedded.

2. Sternum closure device according to claim 1, **characterised in that** the plastics material is polyether ether ketone.

3. Sternum closure device according to claim 1, **characterised in that** the plastics material is a resorbable plastics material.

4. Sternum closure device according to any one of the preceding claims, **characterised in that** the web (17) has through openings (19), through which the plastics material passes.

## Revendications

1. Système de fixation pour sternum (1) destiné à la fixation de deux parties de sternum (22, 23) devant être raccordées, comprenant une plaque de retenue intérieure (2) destinée à reposer sur la surface interne du sternum, au moins un élément de serrage qui y est fixé, s'étendant transversalement à partir de celle-ci et une plaque de retenue extérieure (3) destinée à reposer sur la surface externe du sternum, qui peut être serrée au moyen de l'élément de serrage traversant l'interstice (24) séparant les parties de sternum (22, 23) contre la première plaque de retenue, la plaque de retenue intérieure (2) se composant au moins partiellement d'une matière plastique biocompatible et les parties métalliques étant incorporées dans la matière plastique, **caractérisé en ce que** la plaque de retenue intérieure (2) présente une partie médiane (18) constituée de la matière plastique biocompatible dans laquelle est incorporée une âme (17) en métal, prenant la forme d'une nervure de renforcement et dotée de saillies de retenue (16) pour la fixation de la plaque de retenue intérieure (2) sur le sternum, situés respectivement des deux côtés longitudinaux opposés.

2. Système de fixation pour sternum selon la revendication 1, **caractérisé en ce que** la matière plastique est le polyétheréthercétone.

3. Système de fixation pour sternum selon la revendication 1, **caractérisé en ce que** la matière plastique est une matière plastique résorbable.

4. Système de fixation pour sternum selon l'une des revendications précédentes, **caractérisé en ce que** l'âme (17) présente des ouvertures (19) à travers lesquelles pénètre la matière plastique.
